Europäisches Patentamt

European Patent Office

Office européen des brevets

(11) Publication number: **0 311 163**
**A2**

## (12) EUROPEAN PATENT APPLICATION

(21) Application number: 88202026.6

(22) Date of filing: 16.09.88

(51) Int. Cl.4: **C12N 9/18 , C12Q 1/44 , C12N 1/06**

(30) Priority: 18.09.87 NL 8702231

(43) Date of publication of application:
**12.04.89 Bulletin 89/15**

(84) Designated Contracting States:
**AT BE CH DE ES FR GB GR IT LI LU NL SE**

(71) Applicant: **DUPHAR INTERNATIONAL RESEARCH B.V**
**C.J. van Houtenlaan 36**
**NL-1381 CP Weesp(NL)**

(72) Inventor: **Op Den Kamp, Josephus A.F.**
**c/o Octrooibureau Zoan B.V. P.O. Box 140**
**NI-1380 AC Weesp(NL)**
Inventor: **Slotboom, Arend J.**
**c/o Octrooibureau Zoan B.V. P.O. Box 140**
**NL- 1380 AC Weesp(NL)**
Inventor: **Van Scharrenburg, Gustaaf J.M.**
**c/o Octrooibureau Zoan B.V. P.O. Box 140**
**NL-1380 AC Weesp(NL)**

(74) Representative: **Muis, Maarten et al**
**OCTROOIBUREAU ZOAN B.V. P.O. Box 140**
**NL-1380 AC Weesp(NL)**

(54) Use of site-specific modified lipolytic enzymes for the selective hydrolysis of organized lipid structures.

(57) The invention relates to a method of selectively hydrolysing organized phospholipids by means of lipolytic enzymes having regulated penetrating and hydrolising capacity due to site-specific modification with a hydrophobic group.

More particularly, useful site-specific acylated pancreatic phospholipases $A_2$ are capable of hydrolysing phospholipids of organized lipid-water interfaces, for example micelles, vesicles, liposomes and other bilayer structures, hexagonal $H_{II}$-phases or biological membranes.

The invention also relates to a method for the preparation of these modified enzymes and to the enzymes in substantially pure form.

# Use of site-specific modified lipolytic enzymes for the selective hydrolysis of organized lipid structures.

The invention relates to site-specific modified lipolytic enzymes, in which the modification consists of the specific covalent introduction of a hydrophobic group, as a result of which the penetrating and hydrolysing capability of the enzyme is regulated by means of variation of the hydrophobic character of said group, for example, by variation in structure and length of a hydrocarbon chain.

The invention also relates to the preparation and the use of the said modified enzyme. According to the invention it is possible to use this class of enzymes for the selective penetration and hydrolysis of lipid-water interfaces, for example, micelles, vesicles, liposomes and cell-membranes, on the basis of differences in surface pressure. Morphological changes of cell-membranes as a result of lipid hydrolysis may lead to irreversible loss of membrane function, which ultimately results in cell death. It was found that Plasmodium-infected erythrocytes (malaria) are lysed selectively as compared with non-infected erythrocytes by means of selected acylated phospholipases $A_2$ (PLA's).

Differences in activity and substrate specificity occur within the class of lipolytic enzymes. All the enzymes of this class have in common that they are capable of hydrolysing substrates organized in lipid-water interfaces, for which penetration of the organized lipid structure is an essential condition.

PLA is the best characterized enzyme of this class and serves as a model for the lipolytic activity. (Verger and De Haas, Annual Review of Biophysics and Bioengineering 5, (1976), 77-117; Volwerk and De Haas, Molecular Biology of Lipid-Protein Interactions (1982), Griffith and Jost. Ed. s) pp. 69-149, Wiley, New York).

It has surprisingly been found that the penetrating and hydrolysing properties of PLA can be strengthened and accurately be regulated by providing the enzyme in specific sites with hydrophobic hydrocarbon chains of variable lengths and structures.

It is known that phospholipase $A_2$ catalyses the hydrolysis of the 2-acyl ester bond in naturally occurring 1,2-diacyl-sn-glycero-3-phospholipids. In this hydrolysis which takes place only in the presence of $Ca^{2+}$-ions, 1-acyl-lysophospholipids and free fatty acids are formed.

The two most important sources of phospholipase $A_2$ are the venom of snakes and the mammalian pancreas. There is a great extent of sequence homology between the pancreatic and snake venom enzymes. Moreover, the three-dimensional structures of the two enzymes show much relationship. However, there are also differences. For example, it is known that pancreatic phospholipases are synthesised in the form of a precursor or zymogen, namely as phosphospholipase $A_2$ which comprises a so-called activating peptide as the terminalα-amino group. This activating peptide is split off by trypsin as a result of which the precursor is converted into active phospholipase $A_2$ which is active on organized lipid-water interfaces. Such a precursor is not known of venom phosphilipase $A_2$.

It is also known that there exist differences in molecular morphology of various phospholipids. Natural lecithins, for example, form closely packed bilayer structures, while anionic phospholipids in the presence of $Ca^{2+}$-ions organize themselves in the hexagonal $H_{II}$-phase.

In contrast to most venom phospholipases $A_2$, pancratic phospholipases $A_2$ prove to have only a very weak penetrating capacity on monomolecular lipid interfaces. They are capable of penetrating into less closely packed or micellar structures, but they are not capable of penetrating into the more closely packed lipid-water interfaces of, for example. vesicles, liposomes and other bilayer structures, for example, membranes.

It has been found that pancreatic phospholipases $A_2$ have two functionally and topographically distinct binding sites for lipids. One of them, which is also present in the precursors, is the classical active site to which monomeric substrates are bound and hydrolysed. In addition to this known binding site, the phospholipases $A_2$ have a so-called lipid binding domain which is formed by a three dimensional cluster of hydrophobic and positively charged amino acids at the surface of the enzyme. This lipid binding domain binds the enzyme to organized lipid-water interfaces. The domain contains a number of hydrophobic amino acid side chains situated at the surface in the positions 3, 6, 19, 20, 31 and 69 of the polypeptide chain. Besides the apolar residues in these loci there are at least two positively charged lysines in the positions 10 and 116 of the lipid binding domain (Dijkstra et al, Nature 289 (1981), 604-606).

It has now been found that useful, site-specific acylated pancreatic phospholipases $A_2$ are obtained when the lysines in position 10 or 116 are acylated with fatty acids of different chain lengths. In this manner phospholipases $A_2$ are obtained having improved and regulated lipid binding properties. The coupling of these acyl groups to the $\epsilon$-amine functions of $Lys^{10}$ or $Lys^{116}$ of pancreatic phospholipases $A_2$ reinforces the penetrating capacity in and the hydrolysis of organized lipid-water interfaces by the enzyme. The invention enables the regulation of these properties by the choice of the length, the position and the structure of the introduced acyl chain.

The invention therefore relates to site-specific monoacylated lipolytic enzymes, for example, phospholipase $A_2$ originating from the pancreas of mammals which are capable of hydrolysing phospholipids of organized lipid-water interfaces, for example, micelles, vesicles, liposomes and other bilayer structures, hexagonal $H_{II}$-phases, or biological membranes in a mixed population of organized lipid-water interfaces with a different packing density to a pre-determined surface pressure. The invention also relates to the preparation and the use of the said enzymes.

It is known that a parasitic or viral infection of a cell can cause a change, i.e. a larger fluidity and lower packing density of the outer membrane of the host cell. This means that these infected and also malignant cells with an outher membrane having a greater fluidity can more easily be penetrated and hydrolysed by the modified enzyme according to the invention than non-infected healthy cells.

The site-specific mono-acylated pancreatic phospholipases $A_2$ according to the invention having a controllable penetrating and hydrolysing capacity for membranes may be used, for example, for:

- selective lysis or selective killing of undesired cells or microorganisms with a small packing density of the outer membrane compared with desired other living cells or microorganisms present.
- selective lysis or selective killing of infected or malignant cells which have a less stable membrane with a lower packing density than native cells as a result of the infection or transformation, in the presence of healthy cells (or tissue).
- selective hydrolysis of phospolipids of lipid-containing particles, if necessary, leaving the more densely packed membranes of cells intact.

As concrete examples of possible applications of the modified phospholipases $A_2$ according to the invention may be mentioned:

1. selective lysis of bloodcells, for example, erythrocytes, T-cells, macrophages etc. which are infected by viruses of parasites, as a result of which these pathogens are ultimately killed or can be better controlled. Parasites which can be controlled in this manner are, for example, Plasmodium, Babesia, Theileria or Anaplasma species; viruses, for example, HIV which causes AIDS,

2. selective hydrolysis of membranes of malignant cells in order to kill these, in other words the use as a chemotherapeutic enzyme,

3. selective lysis of infected cells in a cell culture so as to increase the yield of cell-free pathogen, which is relevant in vaccine production,

4. selected lysis of mycoplasma species which have infected a cell culture or which are the cause of a disease, for example, mastitis in cows and goats,

5. the use as a therapeutic in order to improve the digestion in man and animal, optionally as an additive for enzyme compositions based on mammalian pancreatic tissue,

6. the use as a diagnostic
    a. for determining the surface tension of membranes of cells or lipidic particles;
    b. for detecting cell types or lipidic particles having a reduced surface tension.

7. the use as a strong hydrolysing enzyme for biological sample preparation.

The specificity of the site-specific mono-acylated phospholipases $A_2$ with predetermined penetrating capacity can be further improved for therapeutical application by covalent or non-covalent coupling of a so-called "homing device", for example, a chimeric monoclonal antibody or a hormone, so that the acylated enzyme is directed towards the desired cell type or particle ("targetting").

The following methods, in which the abbreviations used have the meanings given hereinafter, are used for determining the enzymatic activity, the lipid binding properties and the penetrating capacity of the site-specific mono-acylated phospholipases $A_2$ belonging to the invention:

-PLA: phospholipase $A_2$

-AMPrec: fully $\epsilon$-amidinated pro-PLA

-AMPA: fully $\epsilon$-amidinated PLA

-t-Boc: tertiary butoxycarbonyl

-For: formyl

-TFA: trifluoroacetic acid

-Tris: tri(hydroxymethyl)amino methane

-DFP: diisopropylfluorophosphate

-DEAE: diethylamino ethyl

-CM: carboxymethyl

-FPLC: fast protein liquid chromatography
-LC: liquid chromatography
-TPCK: L-1-tosylamido-2-phenylethylchloromethyl ketone
-dpm: disintegrations per minute
-DMF: dimethyl formamide
-Pal: palmitoyl
-Lau: lauroyl
-Ol: oleoyl
-Cap: caprinoyl
-Ac: acetyl
-Mal: maleyl
-OSu: hydroxysuccinimide ester
-DNFB: 2,4-dinitrofluorobenzene
-TNBS: trinitrobenzene sulphonic acid
-$diC_8GS$: 1,2-dioctanoyl-sn-glycero-3-sulphate
-$diC_8PC$: 1,2-dioctanoyl-sn-glycero-3-phosphocholine
-Hepes: N-(2-hydroxyethyl)-piperaxine-N-2-ethanesulphonic acid
-DABITC: dimethylamino azobenzene-iso-thiocyanate.

a. Protein concentrations are determined from the absorption at 280 nm using E 1 cm (1%) of 13.0 for porcine and bovine pancreatic ε-amidinated PLA and the mono-acylated analogs thereof.
A value of 8.8 is used for the N-terminal shortened bovine pancreas enzyme fragments missing $Trp^3$.

b. The binding of the PLA's to micellar cis-9-octadecenyl phosphocholine is determined by means of UV absorption difference spectroscopy (De Aranjo et al., Biochem. 18, (1979), 580-586;; and Hille et al., Biochem. 20, (1981), 4068-4073).

c. Monolayer experiments are carried out using the zero order through with two compartments and the surface barostat technique for measuring phospholipid hydrolysis (Verger and De Haas, Chem. Phys. Lipids, 10, (1973), 127-136). Calculation of the induction time (τ) is carried out as described by Verger et al. J. Biol. Chem. 248 (1973), 4023-4034).
Surface tension (π) is measured by means of the plate method by Wilhelmy using a thin platinum plate attached to a Beckman R II C Model LM 600 microbalance.

d. Kinetic measurements with micellar $diC_8PC$ are carried out according to De Haas et al, Biochem. Biophys. Acta, 239, (1971) 252-266.

I. Kinetic characterization of various (mono-acylated) PLA's.

It appears from Table A hereinafter that the various ε-amidinated pancreatic PLA's which are acylated with fatty acids having a long chain (Cap, Lau, Pal and Ol) at Lys[10] or Lys[116] possess a high enzymatic activity with respect to micellar lecithin having a short chain which is comparable to that of the native enzymes. This means that the inherent catalytic activity of these pPLA's is not affected by the site-specific acylation. Although acylation of Lys[116] with acetyl or maleyl results in a lower $V_{max}$, the remaining activity is sufficient to hydrolyse micellar short chain lecithins effectively.

TABLE A

| Enzyme | Vmax [a] micellar L-dioctanoyl-lecithin ($\mu$equiv. min$^{-1}$ mg$^{-1}$) |
|---|---|
| Porcine | |
| PLA | 2200 |
| AMPA | 1650 |
| [$\epsilon$-NH$_2$-Lys$^{116}$]AMPA | 2000 |
| [$\epsilon$-Ac-Lys$^{116}$]AMPA | 755 |
| [$\epsilon$-Mal-Lys$^{116}$]AMPA | 405 |
| [$\epsilon$-Cap-Lys$^{116}$]AMPA | 2100 |
| [$\epsilon$-Lau-Lys$^{116}$]AMPA | 2500 |
| [$\epsilon$-Pal-Lys$^{116}$]AMPA | 2200 |
| [$\epsilon$-Ol-Lys$^{116}$]AMPA | 2600 |
| Bovine | |
| PLA | 3000 |
| AMPA | 2300 |
| [Arg$^6$]-AMPA | 5900 |
| [Arg$^6$,$\epsilon$-Pal-Lys$^{10}$]-AMPA | 5600 |

a) Maximum velocity determined by titration of fatty acid which has been obtained with 10.3 mM NaOH at pH 6.0 (see Van Scharrenburg et al., Biochem. 20, (1981), 1589-1591).

II. Parameters for the binding of various mono-acylated PLA's to micellar cis-9-octadecenyl phosphocholine.

The binding properties of the various PLA's to the single strain micellar substrate analog cis-9-octadecenylphosphocholine are recorded in Table B hereinafter. From the table it appears that the presence of a covalently attached long chain fatty acid on Lys$^{116}$ or Lys$^{10}$ considerably improves the binding of the enzyme to the zwitterionic detergent. A larger chain length of the fatty acid leads to a lower apparent dissociation constant (N.K$_D$), which means a greater affinity to the organized lipid-water interface which is formed by the micellar substrate analog.

Introduction of a cis-double bond in the acyl chain (oleyl = cis-9-octadecenoyl) to Lys$^{116}$ results in a decrease of the affinity to the micelles compared with the [$\epsilon$-Pal-Lys$^{116}$]-enzyme the acyl chain of which is even shorter. Such a cis-double bond gives a bend of the acyl chain which is clearly unfavourable for the binding of the [$\epsilon$-Ol-Lys$^{116}$]-enzyme to organized lipid-water interfaces.

It will become obvious from the results recorded in Table B that the affinity of the acylated enzymes to organized lipid structures depends on the position, structure and length of the acyl chain.

TABLE B

| Enzyme | N.$K_D$[a] ($\mu$M) apparent dissociation constant |
|---|---|
| Porcinepancreatic | |
| PLA | 58 |
| AMPA | 76 |
| [$\epsilon$-NH$_2$-Lys$^{116}$]-AMPA | 44 |
| [$\epsilon$-Ac-Lys$^{116}$]-AMPA | 38 |
| [$\epsilon$-Mal-Lys$^{116}$]-AMPA | 43 |
| [$\epsilon$-Cap-Lys$^{116}$]-AMPA | 33 |
| [$\epsilon$-Lau-Lys$^{116}$]-AMPA | 29 |
| [$\epsilon$-Pal-Lys$^{116}$]-AMPA | 12 |
| [$\epsilon$-Ol-Lys$^{116}$]-AMPA | 18 |
| Bovinepancreatic | |
| PLA | 660 |
| AMPA | 3200 |
| [Arg$^6$]-AMPA | 120 |
| [Arg$^6$,$\epsilon$-Pal-Lys$^{10}$]-AMPA | 20 |
| a) N.$K_D$ = apparent dissociation constant N = number of lipid monomers (cis-9-octadecenylphosphocholine) per molecule of enzyme. $K_D$ = micellar dissociation constant | |

III. The penetrating capacity of the various PLA's for substrate monolayers.

In the monolayer experiments the technical equipment has been used as described by Verger and De Haas, J. Biol. Chem. 248, (1973), 4023-4034, in which the surface pressure related with the packing density of a lipid monolayer spread over the water-air interface is automatically kept constant by the movement of the surface barrier. Lipolytic enzymes which penetrate into such a monolayer consisting of 1,2-didecanoyl-sn-glycero-3-phosphocholine, hydrolyse said substrates into products which dissolve immediately in the water phase. As a result of the enzymatic activity, a continuously recorded movement of the surface barrier is necessary to keep the surface pressure constant. With a particular trough-design (zero-order trough; Verger and De Haas, J. Biol. Chem. 248, (1973), 4023-4034), this movement of the surface barrier in time is directly proportional to the catalytic rate of the reaction (expressed in molecules/cm$^2$/min.).

Another advantage of the use of the "zero-order" trough monolayer technique is the applicability thereof for the determination of the induction time ($\tau$) of a lipolytic enzyme. The induction time is the time which is necessary to penetrate into a monolayer of substrate molecules with a predetermined surface pressure, until the "steady state" situation is reached (Vab Dam-Mieras, thesis 1976, University of Utrecht).

In general, the induction time of PLA's to penetrate a monolayer of lecithin rapidly increases at a certain surface pressure ($\pi$) when the surface pressure of the lipid-water interface further increases. This indicates that the enzyme, from a certain surface pressure, cannot penetrate or can hardly penetrate into a liquid layer and cannot hydrolyse same.

By means of this monolayer technique it is possible to quantify the penetrating capacity of site-specific mono-acylated PLA's in lipid-water interfaces, for example, monolayers, micelles, hexagonal H$_{II}$-phases, vesicles, liposomes, bilayers, and biological membranes.

It has been found that the penetrating capacity of acylated PLA's can be regulated by means of the position, the structure and the length of the acyl chain:

a) the penetrating capacity of acylated PLA's increase with increasing chain length of the acyl chain;

b) acylation of Lys$^{116}$ results in a larger penetrating capacity than corresponding acylation of Lys$^{10}$,

c) acylation with an unsaturated acyl chain or a branched acyl chain results in a mono-acylated enzyme with a lower penetrating capacity than acylation in the same position with a saturated or straight acyl chain with the same number of C-atoms.

This presents the possibility of synthesising acylated PLA's which are capable of penetrating and hydrolysing organized lipid-water interfaces to a predetermined surface pressure, so that such PLA's are suitable for the applications according to the invention mentioned hereinbefore.

The invention also relates to the synthesis of site-specific acylated PLA's.

Porcine and bovine pancreatic pro-PLA's can be purified and converted into the corresponding PLA's according to the method by Nieuwenhuizen et al., Methods Enzymol. 32B, (1974), 147-154, and Dutihl et al. Eur. J. Biochem. 53, (1975), 91-97, respectively.

The pancreatic pro-PLA's are fully ε-amidinated according to the method by Wallace and Harris, Biochem. J., 217, (1984), 589-594, and converted into AMPA according to the method of Slotboom and De Haas, Biochemistry, 14, (1975), 5394-5399.

N-hydroxysuccinimide esters (N-OSu) of caprinic acid, lauric acid, palmitic acid and oleic acid can be obtained as described by Lapidos et al., J. Lipid Res. 8, (1967), 142-145, and be purified by means of crystallisation and/or chromatography over silica gel.

The preparation of 1,2-dioctanoyl-sn-glycero-3-sulphate is described in Biochemistry 24, (1985), 7993-7999.

The site-specific Lys $^{10}$ mono-acylated pancreatic PLA's according to the invention can be obtained according to the reaction scheme below;

Reaction scheme I

Conversion of fully ε-amidinated bovine pancreatic AMPrec in [Arg$^5$, ε-Pal-Lys$^{10}$]-AMPA

```
        -7      -1 ¦ 1       6     7    8    9       10     11        123
      <Glu....Arg¦.Ala....Asn.Gly.Met⌋.Ile.Am-Lys.Cys.....Cys
                 ¦                     ⌐⟶  ⟶
                 ⌄

                        (bovine AMPrec)
```

```
                                        1)CNBr(↓)
      trypsin  (¦)                         2) Edman decomp. (⟶)
               ⌄

      AMPA ↙
```

```
                                    11                    123
                                H₂NCys..............Cys
```

$$1)\underline{N}\text{-}\alpha\text{-}t\text{-}Boc\text{-}\underline{N}\text{-}\varepsilon\text{-}Pal\text{-}Lys$$
$$2)\underline{N}\text{-}\alpha\text{-}t\text{-}Boc\ Ile\text{-}OSu$$
$$3)\underline{N}\text{-}\alpha\text{-}t\text{-}Boc\text{-}Met\text{-}Osu$$

```
                                1                        6      7
      4)N-α-t-Boc.Ala.Leu.Trp(For)Gln.Phe.Arg-Gly
```

```
      1           6     7    8    9      10    11            123
      H₂N-Ala........Arg.Gly.Met.Ile.Pal-Lys.Cys...........Cys
```

$$([Arg^6, Pal\text{-}Lys^{10}]\text{-}AMPA)$$

The preparation of fully amidinated porcine pancreatic PLA with an acyl group at Lys$^{116}$, i.e. [ε-acyl-Lys$^{116}$]- AMPA, can be carried out as shown in reaction scheme II.

Reactionscheme II

Conversion of pro-PLA (i.e. Prec) into [ε-Acyl-Lys$^{116}$]-AMPA

$$\text{Prec} \xrightarrow{\text{di-C}_8\text{GS}} \text{Prec.di-C}_8\text{GS}$$

$$CH_3-C \overset{NH.HCl}{\underset{OCH_3}{\diagup\!\!\!\diagdown}} \qquad\qquad CH_3-C \overset{NH.HCl}{\underset{OCH_3}{\diagup\!\!\!\diagdown}}$$

AMPrec $\qquad\qquad\qquad$ $[\varepsilon\text{-}NH_2\text{-}Lys^{116}]\text{-}AMPrec$

activated fatty acid

trypsin

trypsin

AMPA $\qquad\qquad\qquad$ $[\varepsilon\text{-}acyl\text{-}Lys^{116}]AMPrec$

trypsine

$[\varepsilon\text{-}NH_2\text{-}Lys^{116}\text{-}]AMPA$

$[\varepsilon\text{-}acyl\text{-}Lys^{116}]\text{-}AMPA$

The preparation of $Lys^{10}$ and $Lys^{116}$ mono-acylated AMPA's according to the invention is described in greater detail in Examples I and II hereinafter.

Another possible way of preparing site specific mono-acylated mammalian pancreatic PLA's according to the invention is presented by the recombinant DNA technology.

The porcine pancreatic PLA-gene has been cloned and expressed in E.coli $K_{12}$ and in yeast (see De Geus et al, Nucl. Acids. Res., vol. 15 (1987), 3743-3759). Site-directed mutagenesis offers the possibility to substitute any of the positively charged or neutral amino acid residues which form the lipid binding domain, by a cystein. The PLA analog which contains only one cystein with a free SH group in the position 3, 6, 10, 19, 20, 31, 69 or 116 of the polypeptide chain, may then be converted into the desired mono-acylated lipolytic enzyme with predetermined penetrating capacity according to the invention by acylating the only free SH-group. Many methods are known of acylating free SH-groups which are more reactive than other functional groups which are present in a protein.

In contrast with the above-described methods of preparing $Lys^{10}$ or $Lys^{116}$ mono-acylated lipolytic enzymes, no ε-amidinated PLA is obtained in this manner but mono-acylated (in positions 3, 6, 10, 19, 21, 31, 69 or 116) PLA's with free ε-$NH_2$ groups. As appears from the above tables A and B, the amidino groups in the AMPA's have only a minor influence on the catalytic, lipid-binding and penetrating properties of the mammalian pancreatic PLA's.

Example I

Preparation of bovine pancreatic [ε-Pal-Lys¹⁰,Arg⁶]-AMPA

The ε-amidinated PLA fragments $NH_2-Cys^{11}.....Cys^{123}$ was obtained by CNBr-cleavage at Met[8] (see Van Scharrenburg et al., Biochemistry 20, (1981), 1589-1591) followed by two successive Edman-degradations in oder to remove Ile[9] and amidino-Lys[10].

N-α-t-Boc-N-ε-palmitoyl-lysin was prepared by reaction of palmitoyl-N-hydroxysuccinimide ester with N-α-t-Boc-lys (see Lapidos et al., J. Lipid Res. 8, (1967), 142-145), with the proviso that a mixture of $CH_2Cl_2$-DMF (1:15 vol/vol) was used as a solvent and that N-methylmorpholine was added as a base. After termination of the reaction (24 hours) the mixture was evaporated to dryness under reduced pressure. The residue was recrystallised from $CHCl_3$-petroleum ether at -20°C. In this manner pure N-α-t-Boc-N-ε-palmitoyl-Lys was obtained in a yield of 90%. N-α-t-Boc-N-hydroxysuccinimide esters of L-Met and L-Ile, were obtained from Fluka A.g. (Switzerland). According to the method of Van Scharrenburg, Biochemistry 20, (1981), 1589-1591 N-α-t-Boc-Ala.Leu.Trp(For).Gln-.Phe.Arg.Gly was prepared and purified. N-α-t-Boc-N-ε-palmitoyl-Lys (0.43 g, i.e. a 10-fold molar excess) was coupled to the enzyme fragment by means of the mixed anhydride method (Van Scharrenburg et al). The excess of the reagent was removed by elution of the reaction mixture in two equal portions on a Sephadex LH-20 column (85x3cm) by means of 90% DMF. The protein-containing fractions were combined, dialysed and lyophilized. In order to hydrolyse all the tyrosyl esters the protein was dissolved in a 1 M $NH_2OH$ solution (pH 7.0) and kept at room temperature for 20 minutes. After dialysing the solution was lyophilized. The N-α-t-Boc group was then removed by treatment with TFA followed by lyophilisation. The polypeptide chain was elongated with Boc-Ile and Boc-Met by means of the respective N-hydroxysuccinimide esters.

The t-Boc groups were removed by treatment with TFA. The intermediate product des (Ala¹-Gly⁷)-[ε-Pal-Lys¹⁰]-AMPA was purified on a CM-cellulose column (60x2cm; Whatman), equilibrated with 5mM Na-acetate (pH 6.0) and eluted with 4 litres of a linear salt gradient (up to 0.2M NaCl) in the same buffer (yield 0.4 g).

Finally, N-t-Boc-Ala¹.Leu.Trp(For).Gln.Phe.Arg.Gly was coupled to des (Ala¹-Gly⁷)-[ε-Pal-Lys¹⁰]-AMPA by means of the mixed-anhydride method.

After removing the formyl and t-Boc groups the protein was purified on a DEAE-cellulose column (42x1.5 cm; Whatman), equilibrated with 5mM Tris buffer and eluted with 1 litre of a linear salt gradient (up to 0.07 M NaCl). In this manner 81 mg of pure [ε-Pal-Lys¹⁰,Arg⁶]-AMPA were obtained.

The other Lys¹⁰ mono-acylated PLA's can be obtained in an analogous manner.

EXAMPLE II

Preparation of porcine pancreatic [ε-alyl-Lys¹¹⁶]AMPA's

a) Preparation of [ε-NH₂-Lys¹¹⁶]-AMPrec (ε-amidinated PLA).

Porcine pancreatic PLA (0.5 g) and di-$C_8$GS (0.17 g) were pre-incubated at room temperature in 90 ml of buffer (2.0 mM sodium tetraborate with 5 mM of EDTA; pH 9.5) while stirring magnetically. After 10 minutes a freshly prepared solution of 0.75 g of methylacetimidate hydrochloride in 20 ml of buffer was added. The reaction mixture was stirred at room temperature and the reaction was discontinued after 40 minutes by the addition of acetic acid to pH 3.8. The mixture was then stirred at 4°C for another 30 minutes. The precipitate of the complex with a high molecular weight of partly ε-amidinated pro-PLA and substrate which is formed upon acidification was centrifuged for 30 minutes at 4000 rpm and 40°C.

The clear supernatant liquid contained less than 1% of the original amount of protein and was discarded. The precipitate dissolved in 25 ml of buffer (5 mM Tris with 5 mM $Ca^{2+}$ and 0.4 mM DFP (pH 8.5) was incubated overnight with 5 mg of native porcine PLA to degrade the substrate. After dialysis of the incubation mixture at 4°C against 0.1% acetic acid to remove salt and decomposition products of the substrate, the solution was freeze-dried. In order to obtain pure ε-amidinated pro-PLA's which comprise 0, 1, or 2 or more non-aminidated ε-NH₂-lysyl residues, negatively charged maleyl groups on the non-amidinated lysyl residues were introduced into the partly amidinated PLA and separated by means of ion exchange chromatography. This was carried out by the addition of 10 portions of 0.1 ml of a maleic acid anhydride solution (100 mg of maleic acid anhydride per ml of absolute dioxan) with 2-minute intervals to a magnetically stirred solution of the crude amidinated pro-enzyme (0.25 g) in 25 mol of buffer (0.1 M sodium tetraborate; pH 8.5). During the addition the pH was kept at 8.5 by the addition of a NaOH solution. The reaction mixture was then stirred at room temperature for 45 minutes. In order to split off all maleyl esters from tyrosyl residues an equal volume of a 2 mM $NH_2OH$ solution was added and stirring was continued at room temperature for another 20 minutes. After dialysing against 0.1% acetic acid at $4^\circ$ C for 24 hours, the solution was freeze-dried. The crude maleinated-amidinated pro-PLA was separated by means of chromatography on DEAE-cellulose. Three fractions A, B and C were obtained in yields of 40, 50 and 10%, respectively. These were combined, dialysed and freeze-dried. Fraction A proved to be fully $\epsilon$-amidinated pro-PLA. The fractions B and C, were eluted after A, from the positively charged ion-exchanger by the pressure of one and two, respectively, negatively charged maleyl groups. Both fractions were demaleinated by incubation in a HCl solution at pH 2.5 for 24 hours at $50^\circ$ C (protein concentration 2 mg/ml) (see Butler et al., Biochem. J. 112, (1969), 679-689). After freeze-drying, the demaleinated fractions were purified by chromatography on DEAE-cellulose. The number of free $\epsilon$-$NH_2$-lysyl groups in the fractions B and C was determined by means of the DNFB and TNBS procedures (see Wolfsy et al., Biochem. 2, (1963), 104-116, and Habeeb, Anal. Biochem. 14, (1966), 328-336, respectively). The TNBS content was calculated from the absorption maximum at 345 nm using $E_{345}$ = 1.45 x $10^{-4}$ $M^{-1}$ $cm^{-1}$ (see Okuyama et al., J. Bioohem. (Tokyo) 47, (1960), 454-466).

b) Localisation of the non-amidinated Lys-group(s) in [$\epsilon$-$NH_2$-Lys]-AMPrec.

[$\epsilon$-$NH_2$-Lys]-AMPrec was incubated with 0.1% (wt/wt) trypsin at pH 8.0 (10 mM Tris, 5 mM $Ca^{2+}$) in order to obtain [$\epsilon$-$NH_2$-Lys-]AMPA. This protein was purified on a DEAE-cellulose column (15 x 1 cm), equilibrated with 5 mM Tris pH 7.5 and eluted with 200 ml of linear salt gradient (up to 0.12 M NaCl). After dialysing and freeze-drying, pure [$\epsilon$-$NH_2$-Lys]-AMPA was obtained. Dissolved in 10 mM Tris/HCl and 5 mM $Ca^{2+}$ (pH 8.0) and a protein concentration of 1 mg/ml, this enzyme was incubated with TPCK-trypsin (15% wt/wt) at room temperature. The progress of the reaction was followed by means of FPLC (Mono Q column, Pharmacia). After 3 hours, less than 5% of the enzyme activity proved to be left and incubation was discontinued and the resulting mixture was then separated on a DEAE-cellulose column (38 x 1.5 cm), equilibrated with 5 mM Tris. pH 7.5 and eluted with 0.6 litre of a linear salt gradient (up to 0.4 M NaCl). The first elution peak proved to be pure. Amino acid analysis (according to Spackman et al., Anal. Chem. 30, (1958), 1190-1206) after 24 hours hydrolysing with 5.6 N HCl under reduced pressure at $110^\circ$ C showed that the N-terminal hexapeptide Ala.Leu.Trp.Gln.Phe.Arg.Gly had been obtained in a yield of more than 90%.

Two other fractions peak II and III, upon analysis after desalting on a Sephadex column G-25 (0.1% acetic acid) and freeze-drying, proved to consist of des (Ala$^1$-Arg$^6$)-[$\epsilon$-$NH_2$-Lys]-AMPA and were obtained in yields of 30% and 70%. After renewed incubating the peak II-fraction with TPCK-trypsin, this fraction proved to have passed to peak III almost quantitatively. The fraction of peak III was subjected to Edman degradation by means of the DABITC-procedure (see Chang, Meth. Enzymol, 91 (1983), 455-466). As a result Ser + Asn, Met + Leu and Ile + Asp. respectively, could be identified. Apparently, Ser, Met and Ile originate from the positions 7, 8 and 9 after cleavage of the N-terminal hexapeptide Ala$^1$- Arg$^6$. The sequence Asn.Leu.Asp occurs twice in the porcine enzyme, in both cases preceded by a Lys (the positions 57, 58, 59 and 117, 118, 119). In order to be able to distinguish between these two possibilities, fraction of peak III was oxidised with performic acid (Hirs, Meth. Enzymol. 11, (1967), 197-199), and after freeze-drying the mixture was separated on a Sephadex G-25 column (70 x 2 cm), equilibrated with 0.05 M $NH_4HCO_3$, pH 8.0. Two peaks were obtained: one eluted in the "void" volume. ($C_I$) and one later ($C_{II}$). After amino acid analysis, fraction $C_I$ proved to be the peptide 7-116. Fraction $C_{II}$ which was found to be pure on LC, gave the following picture after amino acid analysis: Asx = 2.0; Thr = 1.0, cysteic acid = 0.9; Leu = 1.0; Lys + $\epsilon$-amidino-Lys = 1.7; Tyr = +. This analysis is in good agreement with the C-terminal sequence Asn$^{117}$.-Leu.Asp.Thr.$\epsilon$-amidino-Lys.$\epsilon$-amidino.Lys.Tyr.Cys$^{124}$. Both the peptide 7-116 ($C_I$) and the peptide 117-124 ($C_{II}$) were obtained in a yield of 85%. It appears unambiguously from these results that the free lysin which is present in [$\epsilon$-$NH_2$-Lys]-AMPrec is located at position 116.

c) Preparation of porcine pancreatic [$\epsilon$-acyl-Lys$^{116}$]-AMPA's.

EP 0 311 163 A2

18 mg Of (1-$^{14}$C) palmitoyl-N-hydroxy-succinimide ester (10-fold molar excess; 0.315 x 10$^6$ ppm/$\mu$mol) dissolved in 2.5 ml of DMF were added to a magnetically stirred solution of [$\epsilon$-NH$_2$-Lys$^{116}$]-AMPrec (70 mg) in 35 ml of a mixture of DMF and 0.02 M Hepes buffer (pH 8.3), (9:1; vol/vol). The reaction mixture was kept at room temperature and after 8 hours another 18 mg of the ester in 2.5 ml of DMF were added, after which the reaction was continued overnight at room temperature. To remove the excess of reagent, the reagent mixture was provided on a Sephadex LH-20 column (95 x 2.5 cm; flow rate 18 ml/hour) and eluted with 50% DMF. The protein-containing fractions were combined, dialysed against 0.1% acetic acid, and freeze-dried. To hydrolyse the palmitoyl esters of tyrosin, a solution of the acylated protein (2 mg/ml) was treated at room temperature for 20 minutes with an equal volume of 2 M NH$_2$OH (pH 7.5). After thorough dialysis at 4$^\circ$C against 0.1% acetic acid the solution was lyophilized, yielding the desired [(1-$^{14}$C)-$\epsilon$-Pal-Lys$^{116}$]AMPrec in a yield of 93%. The specific radioactivity was found to be 0.296 x 10$^6$ ppm/ $\mu$mol, which indicates the take up of 0.94 palmitoyl residues per molecule of protein, which is in good agreement with the values which have been obtained in the DNFB- and TNBS-procedures for [$\epsilon$-NH$_2$-Lys$^{116}$]-AMPrec. FPLC demonstrated that the acylated protein is pure (Mono Q and S columns of Pharmacia with mixtures (1:1; vol/vol) of buffer and acetonitrile). Dissolved in 5 mM Tris-HCl buffer (pH 8.0) with 5 mM CaCl$_2$, [(1-$^{14}$C)-$\epsilon$-Pal-Lys$^{116}$]-AMPrec was activated by means of 0.1% (wt/wt) trypsin as a result of which [(1-$^{14}$C)-$\epsilon$-Pal-Lys$^{116}$]-AMPA was obtained.

[(1-$^{14}$C)-$\epsilon$-Lau-Lys$^{116}$]-, [$\epsilon$-Cap-Lys$^{116}$]- and [$\epsilon$-Ol-Lys$^{116}$]-AMPrec and -AMPA were obtained in an analogous manner. The specific radioactivity of (1-$^{14}$C)-lauroyl-N-succinimide ester was 1.02 x 10$^6$ ppm/ $\mu$mol and that of [(1-$^{14}$C)-$\epsilon$-Lau-Lys$^{116}$[-AMPrec 0.93 x 10$^6$ ppm/ $\mu$mol, which indicates the take up of 0.91 lauroyl residues per molecule of protein.

d) The preparation of porcine pancreatic [(1-$^{14}$C)-$\epsilon$-Acetyl-Lys$^{116}$]-AMPA.

0.5 ml Of (1-$^{14}$C) acetic acid anhydride (6.24 x 10$^6$ ppm/ $\mu$mol in 2 ml of dry dioxan was added in five portions at room temperature in 30 minutes to a magnetically stirred solution of 25 mg of [$\epsilon$-NH$_2$-Lys$^{116}$]-AMPrec in 5 ml of 0.5 M sodium tetraborate (pH 10). The pH was kept at 10 by the addition of a 1.5 N NaOH solution. After everything had been added, the mixture was stirred at room temperature for another 20 minutes. An equal volume of a 2 M NH$_2$OH solution (pH 7.5) was then added and the mixture was stirred at room temperature for 20 minutes. The reaction mixture was desalted by elution on a Sephadex G-25 column (0.1% acetic acid) and the protein fractions were combined, dialysed against 0.1% acetic acid, and lyophilized. The acylated AMPrec was finally purified chromatographically on a DEAE-cellulose column (10 x 1.5 cm), equilibrated with 5 mM Tris at pH 7.5 and developed with 0.3 litres of a linear salt gradient (up to 0.25 M NaCl). Fractions containing [(1-$^{14}$C)-$\epsilon$-Acetyl-Lys$^{116}$]-AMPrec were combined, dialysed at 4$^\circ$C against 0.1% acetic acid and lyophilized. 19 mg Of pure [(1-$^{14}$C)-$\epsilon$-Acetyl-Lys$^{116}$]-AMPrec were obtained in this manner with a specific radioactivity of 3.69 x 10$^6$ ppm/ $\mu$mol, which indicates the presence of 1.18 acetyl groups per molecule of protein. Activation with trypsin was carried out in the manner described hereinbefore, after which the [(1-$^{14}$C-$\epsilon$-Acetyl-Lys$^{116}$]-AMPA was obtained.

EXAMPLE III

Porcine pancreatic -$\epsilon$-amidinated PLA[$\epsilon$-Lau-Lys$^{116}$] and bovine pancreatic $\epsilon$-amidinated PLA (Arg$^6$, $\epsilon$-Pal-Lys$^{10}$], enzymes which are not capable of lysing non-infected erythrocytes, were used for the in vitro lysis of erythrocytes infected with Plasmodium knowlesii (a malaria parasite). The following general techniques were used:
- Plasmodium knowlesii (ring stage) infected and non-infected erythrocytes were separated by means of centrifuging using a gradient of Percoll/sorbitol. Non-infected cells shrivelled and diffuse in the gradient, while infected cells are permeable to sorbitol and remain in the upper layer. The gradient had the following composition:

upper layer = PBS/sorbitol + cells    2 ml
6.66 vol. SIP + 3.33 vol. PBS/sorbitol    2 ml
7.77 bol SIP + 2.22 vol. PBS/sorbitol    2 ml
8.88 vol. SIP + 1.11 vol. PBS/sorbitol    3 ml
SIP    4 ml
PBS/sorbitol = PBS with 6 g of sorbitol/100 ml
SIP - 1 vol. 1.5 NaCl + 1 vol. of 6 g of sorbitol/100 ml

The cells were centrifuged at 1,000 rpm, collected and washed a few times so as to remove any remaining sorbitol.

- 5% suspensions of infected and non-infected cells were incubated, while shaking gently, with 8.5 IU (international units) per ml of site-specific mono-acylated, $\epsilon$-amidinated PLA's at 37° C in a buffer which contains 140 mM of NaCl, 10 mM of $CaCl_2$, 0.25 mM of $MgCl_2$ and 10 mM of Tris-HCl; pH = 7.5. The enzymatic reaction was stopped by the addition of 0.5 ml of 100 mM EDTA-solution to 1 ml of the incubated suspension. After incubation, the cells were centrifuged at 1,500 rpm for 5 minutes. In order to measure the lysis, the extinction of the supernatant was determined at 418 nm.

- In order to measure the extent of hydrolysis of phospholipids the erythrocyte membranes were extracted according to Rose et al., J. Lipid Res. 6, (1965), 428-431. The phospholipids were separated by means of two-dimensional thin layer chromatography according to Broekhuijse, Clin., Chim. Acta 23, (1969), 457-463, and the amount of phosphate was determined according to the method of Rouser et al, Lipids 5, (1970), 494-496.

From Table C hereinafter it appears that of the synthesized PLA derivatives porcine pancreatic [$\epsilon$-Pal-Lys$^{116}$]-AMPA so far has the strongest penetrating capacity for membrane. This enzyme easily attacks the biological membranes of the erythrocyte. In man, the erythrocyte has a lateral surface pressure of 31-35 dyne/cm. The lecithins and their phospholipids which are present in the outer monolayer of the erythrocyte membrane are easily hydrolysed as a result of which the red bloodcells are transformed to so-called echinocytes and subsequent lysis. On the other hand, incubation of erythrocytes with native pancreatic enzymes does not cause any hydrolysis of the membrane lipids. Those native PLA's can penetrate only in organized lipid-water interfaces with a very low surface pressure (10-12 dyne/cm). It has been found possible to make a whole series of mono-acylated PLA's between these extremes with different, intermediate penetrating properties.

TABLE C

| Selective activity of mono-acylated PLA's on the lysis of erythrocytes infected with Plasmodium knowlesii compared with non-infected erythrocytes originating from monkeys. | | | | |
|---|---|---|---|---|
| ENZYME | % lysis of infected cells after ... min. | | | |
| | 10 | 20 | 30 | 60 |
| Porcine pancreatic [$\epsilon$-Lau-Lys$^{116}$]-AMPA | 5.7 | 10.9 | 34.0 | 91.2 |
| Bovine pancreatic [Arg$^6$,$\epsilon$-Pal-Lys$^{10}$]-AMPA | 7.6 | 13.2 | 41.1 | 75.6 |
| | % lysis of non-infected cells after ... min. | | | |
| | 10 | 20 | 30 | 60 |
| Porcine pancreatic [$\epsilon$-Lau-Lys$^{116}$]-AMPA | 3.2 | 4.1 | 4.9 | 6.1 |
| Bovine pancreatic [Arg$^6$,$\epsilon$-Pal-Lys$^{10}$]-AMPA | 2.4 | 2.5 | 3.4 | 4.7 |

It appears from Tables D and E hereinafter that porcine pancreatic [$\epsilon$-Lau-Lys$^{116}$]-AMPA and bovine pancreatic [Arg$^6$, $\epsilon$-Pal-Lys$^{10}$]-AMPA hardly attack and hydrolyse the membranes of normal erythrocytes, whereas the distorted membrane of infected erythrocytes is degraded effectively. Approximately 85-90% of the phosphilipids of membranes of the infected erythrocytes are hydrolysed within 30 minutes under the influence of the strong catalytic activity of the mono-acylated lipolytic enzymes according to the invention.

TABLE D

| Hydrolysis (in %) of phospholipides of infected (A) and non-infected (B) erythrocytes by porcine pancreatic [$\epsilon$-Lau-Lys$^{116}$]-AMPA after ... min. | | | | | | | | |
|---|---|---|---|---|---|---|---|---|
| Phospholipid | 10 | | 20 | | 30 | | 60 | |
| | A | B | A | B | A | B | A | B |
| PC | 28 | 5 | 75 | 15 | 76 | 12 | 86 | 17 |
| PE | 11 | 10 | 87 | 11 | 88 | 8 | 93 | 16 |
| PS/PI | -a) | - | - | - | 87 | - | 84 | - |
| PC = Phosphatidyl choline | | | | | | | | |
| PE = Phosphatidyl ethanolamine | | | | | | | | |
| PS = Phosphatidyl serine | | | | | | | | |
| PI = phosphatidyl inositol | | | | | | | | |

a) = not determined

TABLE E

| Hydrolysis (in %) of phospholipids of infected (A) and non-infected (B) erythrocytes by bovine pancreatic [$Arg^6$,-Pal-Lys$^{10}$]-AMPA after ... min. | | | | | | | | |
|---|---|---|---|---|---|---|---|---|
| Phospholipid | 10 | | 20 | | 30 | | 60 | |
| | A | B | A | B | A | B | A | B |
| PC | 26 | 10 | 89 | 15 | 76 | 16 | 81 | 15 |
| PE | 17 | 2 | 92 | 7 | 91 | 11 | 91 | 16 |
| PS/PI | -a) | - | - | - | 84 | - | 87 | - |
| PC = Phosphatidyl choline | | | | | | | | |
| PE = Phosphatidyl ethanolamine | | | | | | | | |
| PS = Phosphatidyl serine | | | | | | | | |
| PI = phosphatidyl inositol | | | | | | | | |

a) = not determined

### EXAMPLEIV

Porcine pancreatic [$\epsilon$-Lau-Lys$^{116}$]-AMPA is used to inhibit completely in vitro the growth of the malarial parasite Plasmodium falciparum in an infected sample of human erythrocytes.

The following methodology was applied:

- 5 $\mu$l human erythrocytes, 0.5-1.5% infected with Plasmodium falciparum, are suspended in 200 $\mu$l growth medium (RPMI)

- Gibco, 25 mM Hepes, 24 mM $NaHCO_3$/10% human serum AB$^+$.

Subsequently the following amounts of [ -Lau-Lys$^{116}$]-AMPA and AMPA were added to 200 $\mu$l suspensions:

IU [$\epsilon$-Lau-Lys$^{116}$]-AMPA:

0.00121; 0.00484; 0.0121; 0.0484; 0.121; 0.363; 0.847.

IU AMPA:

0.111; 1.11; 1.49; 1.99; 2.41; 2.93; 5.86.

After 24 hours the medium is refreshed and 0.5 $\mu$Ci $^3$H hypoxanthine (14.3 Ci/mmol) is added. The labelled hypoxanthine is incorporated in the DNA of the parasite. In contrast no DNA-synthesis takes place in healthy uninfected erythrocytes. Therefore the amount of radioactivity present in the DNA is a reliable standard of the survival of the parasite.

After 24 to 36 hours of incubation, cells are lysed and the DNA is collected in a filter. Subsequently the radioactivity bound to the filter is measured.

Also the radio-activity is determined of control samples which

    a) were not incubated with enzyme

    b) did not contain infected erythrocytes (back ground measurement)

Good growth of the parasite and the absence of contaminations was further more checked by developing a swab of cells from control dishes with 10% giemsa in phosphatebuffer (pH 7.2).

Incubation of the erythrocytes with the lipolytic enzymes was carried out as described in Example III.

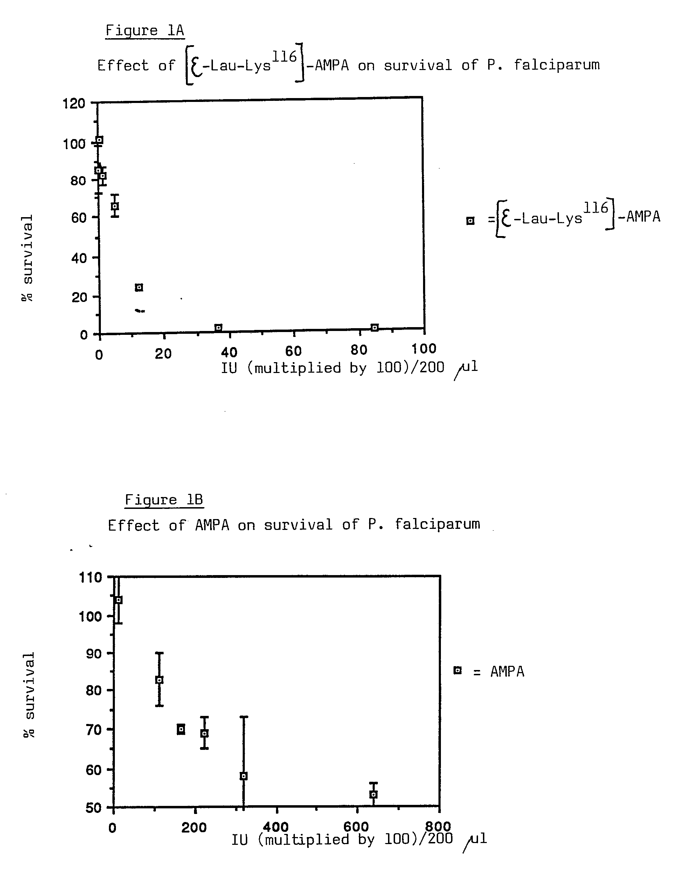

As shown in figure 1A as less as 1.8 IU of [ -Lau-Lys$^{116}$]-AMPA per ml inhibits the growth of the malarial parasite completely. At concentrations of about 0.3 IU per ml 50% inhibition is found. In contrast AMPA inhibits the parasite growth for 50% at much higher concentrations:

29 IU/ml as shown in figure 1B. So in comparison with AMPA the [$\epsilon$-Lau-Lys$^{116}$]-AMPA is about 100 times more effective in combatting the infection.

## Claims

1. A method of hydrolysing phospholipids, characterized in that organized phospholipids are selectively hydrolysed by means of lipolytic enzymes with regulated penetrating and hydrolysing capacity due to site-specific modification with a hydrophobic group.

2. A method as claimed in Claim 1, characterized in that cells or microorganisms, are lysed or killed selectively.

3. A method as claimed in Claim 1, characterized in that infected or malignant cells are lysed or killed in the presence of healthy cells or tissue.

4. A method as claimed in Claim 1, characterized in that phospholipids of lipid-containing particles are hydrolysed selectively.

5. A method as claimed in Claim 1, characterized in that infected bloodcells such as erythrocytes, T-cells or macrophages are killed by selective lysis.

6. A method as claimed in Claim 1, characterized in that cell-free pathogen is prepared by lysing infected cells selectively.

7. A method as claimed in Claim 1, characterized in that mycoplasma cells are killed by selective lysis in the presence of other microorganisms or cells.

8. A method as claimed in Claim 1, characterized in that the surface pressure of membranes of cells or lipid particles is determined for diagnostic purpose, or for tracing cell types or lipid particles having a reduced surface pressure.

9. A method as claimed in Claim 1, characterized in that pure biological samples are opened.

10. A method as claimed in Claims 1-9, characterized in that as modified lipolytic enzyme a site-specific modified phospholipase is used.

11. A method as claimed in Claims 1-9, characterized in that a site-specific acylated phospholipase A$_2$ - (PLA) is used.

12. A method as claimed in Claims 1-9, characterized in that a Lys$^{10}$ or Lys$^{116}$ monoacylated PLA is used.

13. A method as claimed in Claims 1-9, characterized in that a [$\epsilon$-acyl-Lys$^{10}$]-AMPS or [$\epsilon$-acyl-Lys$^{116}$]-AMPA is used.

14. A method as claimed in Claims 1-9, characterized in that a bovine pancreatic [Arg$^6$,$\epsilon$-acyl-Lys$^{10}$]-AMPA or a porcine pancreatic [$\epsilon$-acyl-Lys$^{116}$]-AMPA is used.

15. A method of preparing PLA's, characterized in that

a) fully $\epsilon$-amidinated prophospholipase (AMPrec) is converted chemically or enzymatically into N-terminal amidinated phospholipase (AMPA) N-terminal maximally shortened up to Cys[11],

b) from the resulting AMPA a Lys- or mono-acylated Lys-AMPA is prepared N-terminally in position 3, 6 or 10 in a manner known per se from the peptide preparation, and

c) optionally, Lys is mono-acylated in position 3, 6 or 10.

16. A method of preparing PLA's as claimed in Claim 15, characterized in that

a) Lys[116] in prophospholipase is protected by means of 1,2-dioctanoyl-sn-glycero-3-sulphate (di-$C_8$GS),

b) the resulting complex is amidinated,

c) the partly amidinated complex is treated with PLA to degrade the substrate di$C_8$GS,

d) the pure [$\epsilon$-NH$_2$-Lys[116]]-AMPrec is obtained by maleination of the mixture, separation by ion-exchange chromatography, and subsequent demaleination,

e) the [$\epsilon$-NH$_2$-Lys[116]]-AMPrec thus obtained is mono-acylated by means of an activated fatty acid, and

f) the resulting [$\epsilon$-acyl-Lys[116]]-AMPrec is converted by means of trypsin into [$\epsilon$-acyl-Lys[116]]-AMPA.

17. Modified lipolytic enzymes, characterized in that they are enzymes site-specific modified with a hydrophobic group with regulated penetrating and hydrolysing capacity.

18. Enzymes as claimed in Claim 17, characterized in that they are phospholipases modified site-specific with a hydrophobic group.

19. Enzymes as claimed in Claim 18, characterized in that they are site-specifically acylated phospholipases A$_2$ (PLA's).

20. PLA's as claimed in Claim 19, characterized in that they are Lys[10] or Lys[116] monoacylated PLA's.

21. PLA's as claimed in Claim 20, characterized in that they are [$\epsilon$-acyl-Lys[10]]-AMPA's and [$\epsilon$-acyl-Lys[116]]-AMPA's.

22. PLA's as claimed in Claim 21, characterized in that they are bovine pancreatic [Arg[6], $\epsilon$-acyl-Lys[10]]-AMPA's.

23. PLA's as claimed in Claim 21, characterized in that they are porcine pancreatic [$\epsilon$-acyl-Lys[116]]-AMPA's.

Figure 1A

Effect of $\left[\mathcal{E}\text{-Lau-Lys}^{116}\right]$-AMPA on survival of P. falciparum

$\blacksquare$ = $\left[\mathcal{E}\text{-Lau-Lys}^{116}\right]$-AMPA

Figure 1B

Effect of AMPA on survival of P. falciparum

$\blacksquare$ = AMPA

DUPHAR INTERNAITONAL RESEARCH B.V.